# FASCICULE DE BREVET EUROPEEN

(11) **EP 3 218 715 B1**
(45) Date de publication et mention de la délivrance du brevet: **23.03.2022**
(21) Numéro de dépôt: 15801916.6
(22) Date de dépôt: 10.11.2015
(51) Int. Cl.: G01N 33/558, A61B 5/00, G01N 33/53, G16H 10/40, G16B 99/00, G16B 25/00, G16B 50/30

(54) **SYSTÈME POUR L'ÉVALUATION D'AU MOINS UN ANALYTE SUSCEPTIBLE D'ÊTRE CONTENU DANS UN ÉCHANTILLON LIQUIDE DÉPOSÉ SUR UN DISPOSITIF IMMUNOCHROMATOGRAPHIQUE**
SYSTEM ZUR AUSWERTUNG VON ZUMINDEST EINEM, WAHRSCHEINLICH IN EINER FLÜSSIGKEITSPROBE ENTHALTENEN ANALYTEN AUF EINER IMMUNOCHROMATOGRAFISCHEN VORRICHTUNG
SYSTEM FOR THE EVALUATION OF AT LEAST ONE ANALYTE LIKELY TO BE CONTAINED IN A LIQUID SAMPLE DEPOSITED ON AN IMMUNOCHROMATOGRAPHIC DEVICE

(30) Priorité: 10.11.2014 FR 1460852
(43) Date de publication de la demande: 20.09.2017
(73) Titulaire: NG Biotech, 35480 Guipry (FR)
(72) Inventeur: STANKOV, Milovan, 44630 Plesse (FR); STANKOV-PUGES, Milovan, 35000 Rennes (FR)
(74) Mandataire: Jacobacci Coralis Harle
(86) Numéro de dépôt international: PCT/FR2015/053042
(87) Numéro de publication internationale: WO 2016/075405

(56) Documents cités:
- US-A1- 2005 203 353
- US-A1- 2006 222 567
- US-A1- 2013 203 043
- US-A1- 2013 273 528
- DAVID J. YOU ET AL: "Cell-phone-based measurement of TSH using Mie scatter optimized lateral flow assays", BIOSENSORS AND BIOELECTRONICS, vol. 40, no. 1, 1 février 2013 (2013-02-01), pages 180-185, XP055144932, ISSN: 0956-5663, DOI: 10.1016/j.bios.2012.07.014
- DAE-SIK LEE ET AL: "A simple and smart telemedicine device for developing regions: a pocket-sized colorimetric reader", LAB ON A CHIP, ROYAL SOCIETY OF CHEMISTRY - CAMBRIDGE, GB, vol. 11, no. 1, 7 janvier 2011 (2011-01-07), pages 120-126, XP007917465, ISSN: 1473-0197, DOI: 10.1039/C0LC00209G
- ONUR MUDANYALI ET AL: "Integrated rapid-diagnostic-test reader platform on a cellphone", LAB ON A CHIP, vol. 12, no. 15, 1 janvier 2012 (2012-01-01), page 2678, XP055058669, ISSN: 1473-0197, DOI: 10.1039/c2lc40235a
- ANDRES W MARTINEZ ET AL: "Simple Telemedicine for Developing Regions: Camera Phones and Paper-Based Microfluidic Devices for Real-Time, Off-Site Diagnosis", ANALYTICAL CHEMISTRY, AMERICAN CHEMICAL SOCIETY, US, vol. 80, no. 10, 15 August 2008 (2008-08-15), pages 3699-3707, XP008148137, ISSN: 0003-2700, DOI: 10.1021/AC800112R [retrieved on 2008-04-11]
- Le Poool: "Paroles d'entrepreneur : Milovan Stankov-Puges, pdg de NG Biotech", Youtube, 7 April 2014 (2014-04-07), page 3 pp., XP054981420, Retrieved from the Internet: URL:https://www.youtube.com/watch?v=b--QmS 9Rus0 [retrieved on 2021-02-17]

## Description

### DOMAINE TECHNIQUE AUQUEL SE RAPPORTE L'INVENTION

La présente invention concerne un système pour l'évaluation qualitative et/ou quantitative d'au moins un analyte susceptible d'être contenu dans un échantillon liquide déposé sur un dispositif immunochromatographique.

### ARRIERE-PLAN TECHNOLOGIQUE

La plupart des techniques pour la détermination d'analyte(s) ont progressivement évoluées vers des dispositifs de plus en plus simples d'utilisation, permettant le développement de méthodes de diagnostic de routine, rapides et d'un coût modéré.

Cette évolution a été particulièrement sensible dans le domaine médical, avec l'émergence du diagnostic par « point of care » ou « home testing », dans lequel un diagnostic est directement réalisé près du lit du patient ou à son domicile, sans qu'il soit nécessaire de faire appel aux techniques automatisées de laboratoire d'analyse.

Les immuno-essais font partie des technologies utilisées pour ce type de diagnostic rapide.

Ces immuno-essais regroupent les dispositifs et méthodes de diagnostic basés sur des réactions de liaisons par affinité entre membres de paires de liaisons spécifiques.

Globalement, ces immuno-essais sont divisés en deux grandes approches bien connues.

Dans l'approche dite « compétition », l'analyte recherché et un réactif de détection marqué sont en compétition pour se lier spécifiquement à un réactif de capture. La présence ou l'absence de l'analyte recherché dans l'échantillon sont mesurées, respectivement, par l'absence ou par la présence d'un signal visible (ou mesurable) au niveau du réactif de capture.

Dans l'approche dite « sandwich », un réactif de détection marqué se lie à l'analyte recherché, ce dernier étant immobilisé sur le support solide par l'intermédiaire du réactif de capture. La présence ou l'absence de l'analyte dans l'échantillon liquide sont mesurées, respectivement, par la présence ou par l'absence d'un signal visible (ou mesurable) au niveau du réactif de capture.

Parmi ces immuno-essais, il est connu des dispositifs dits « immunochromatographiques » qui se prêtent particulièrement à certains analytes.

L'immunochromatographie consiste en une méthode de diagnostic en phase solide, utilisant la chimie sèche et la migration latérale sur une membrane inerte.

Ce type d'immuno-essai met en œuvre un support poreux sous forme de bandelette, dans et/ou sur lequel sont intégrés sous forme sèche les réactifs nécessaires à la réalisation du test.

Le support poreux est traité de façon à ce qu'une réaction de reconnaissance entre partenaires de liaison spécifique (généralement antigène / anticorps) se produise au niveau d'une zone de capture et puisse être révélée à ce niveau.

En pratique, lorsque l'échantillon liquide à analyser est déposé sur le support, celui-ci migre le long de la membrane par un phénomène de capillarité. L'analyte recherché est généralement capturé par un réactif de capture spécifique immobilisé sur une zone déterminée de la membrane.

La réaction est révélée par un réactif de détection spécifique marqué, selon le principe de la méthode sandwich.

Il est également possible d'utiliser une réaction de type compétition. On utilise dans ce cas généralement un réactif de détection consistant en un analyte marqué qui est compétiteur de l'analyte recherché pour la liaison spécifique avec le réactif de capture immobilisé.

Ces dispositifs immunochromatographiques sont généralement adaptés à un usage unique et domestique. En effet, ils sont d'un usage facile et rapide, ne nécessitant que très peu de manipulations puisque tous les réactifs sont intégrés ou compris dans le dispositif.

Toutefois, certains dispositifs immunochromatographiques complexes sont susceptibles de donner des résultats dont l'interprétation à l'œil nu est source d'incertitude, voire même impossible.

Pour cette raison, il existe un besoin de dispositifs pour l'évaluation qualitative et/ou quantitative d'au moins un analyte susceptible d'être contenu dans un échantillon liquide déposé sur un dispositif immunochromatographique.

Ces dispositifs d'évaluation comprennent pour cela :
- des moyens pour la réception dudit dispositif immunochromatographique,
- des moyens pour l'acquisition d'au moins une image numérique de la zone de révélation dudit dispositif immunochromatographique rapporté dans lesdits moyens de réception,
- des moyens pour le traitement de ladite au moins une image numérique, adaptés à l'évaluation qualitative et/ou quantitative dudit au moins un analyte susceptible d'être contenu dans ledit échantillon liquide, et
- des moyens pour la fourniture du résultat de ladite évaluation qualitative et/ou quantitative.

De tels dispositifs d'évaluation sont par exemple décrits dans les documents Martinez et al., « Simple Telemedicine for Developing Régions : Camera Phones and Paper-Based Microfluidic Devices for Real-Time, Off-Site Diagnosis », Analytic Chemistry, vol. 80, no. 10, 15 août 2008 (2008-08-15), pages 3699-3707, ou encore dans le document US-2005/203353.

Mais, en pratique, un tel dispositif d'évaluation est particulièrement complexe et onéreux, constituant un obstacle majeur à son déploiement à grande échelle.

De plus, la mise au point d'un tel dispositif d'évaluation est particulièrement complexe, notamment en raison des contraintes de compatibilités entre l'ensemble de ses composants.

En outre, une telle structure oblige l'établissement de données de calibrage dédiées à chaque dispositif d'évaluation, ce qui peut s'avérer là encore complexe et onéreux.

Or, d'une manière générale, de tels systèmes ne sont pas totalement fiables et sécurisés, notamment du fait que les analyses sont effectuées sur un dispositif d'analyse en local.

De tels systèmes d'évaluation peuvent également être limitant lorsque les résultats doivent être regroupés, notamment dans le cadre d'essais cliniques.

Il existe par conséquent un besoin d'un système d'évaluation qui serait particulièrement simple de conception et de prix abordable, de sorte à pouvoir le proposer largement aux patients, notamment pour un suivi à domicile dans le cadre par exemple d'une convalescence, d'une maladie chronique ou d'un essai clinique, avec une fiabilité et une sécurité accrues.

### OBJET DE L'INVENTION

La présente invention concerne ainsi un système pour l'évaluation qualitative et/ou quantitative d'au moins un analyte susceptible d'être contenu dans un échantillon liquide déposé sur un dispositif immunochromatographique, ledit dispositif immunochromatographique comportant des moyens de diffusion capillaire sur lesquels ledit échantillon liquide est destiné à migrer depuis une zone amont de dépôt jusqu'à une zone aval de révélation.

Et conformément à la présente invention, ledit système d'évaluation comprend au moins un dispositif de lecture, un dispositif de transmission et au moins un dispositif d'analyse, qui sont distincts structurellement les uns des autres.

Ledit au moins un dispositif de lecture, formant un appareil portatif, comporte :
- des moyens pour la réception dudit dispositif immunochromatographique, comportant un emplacement pour le positionnement et le maintien du dispositif immunochromatographique,
- des moyens pour l'acquisition d'au moins une image numérique de la zone de révélation dudit dispositif immunochromatographique rapporté dans lesdits moyens de réception, lesquels moyens d'acquisition comprennent un capteur photographique,
- des moyens de communication qui sont adaptés à l'échange de données avec au moins le dispositif de transmission, pour la transmission de ladite au moins une image numérique depuis ledit dispositif de lecture,
- des moyens pour l'éclairage de la zone de révélation du dispositif immunochromatographique,
- des moyens pour l'alimentation autonome en énergie électrique, comprenant une batterie électrique,
lequel au moins un dispositif de lecture est dépourvu :
- de moyens de traitement de ladite au moins une image numérique, adaptés à l'évaluation qualitative et/ou quantitative dudit au moins un analyte susceptible d'être contenu dans ledit échantillon liquide, et
- de moyens pour la fourniture du résultat d'évaluation qualitative et/ou quantitative.

Et ledit au moins un dispositif d'analyse comprend :
- des moyens de communication qui sont adaptés à l'échange de données, pour la réception de ladite au moins une image numérique, lesquels moyens de communication du dispositif d'analyse se présentent sous la forme de moyens de télécommunication, pour la connexion et l'échange de données via le réseau de télécommunication,
- des moyens pour le traitement de ladite au moins une image numérique, adaptés à l'évaluation qualitative et/ou quantitative dudit au moins un analyte susceptible d'être contenu dans ledit échantillon liquide, et
- des moyens pour la fourniture du résultat de ladite évaluation qualitative et/ou quantitative.

Et ledit dispositif de transmission, pour transmettre via un réseau de télécommunication ladite au moins une image numérique acquise depuis ledit au moins un dispositif de lecture jusqu'audit au moins un dispositif d'analyse situé à distance, comprend :
- des moyens de communication qui sont adaptés à l'échange de données avec ledit dispositif de lecture, pour la transmission de ladite au moins une image numérique depuis ledit dispositif de lecture jusqu'audit dispositif de transmission, lesquels moyens de communication équipant le dispositif de lecture et le dispositif de transmission consistent en des moyens de connexion sans fils et courte distance,
- des moyens de télécommunication, pour la connexion et l'échange de données via le réseau de télécommunication, lesquels moyens de télécommunication sont adaptés pour la transmission de ladite au moins une image numérique jusqu'au dispositif d'analyse.

Une telle architecture du système d'analyse a l'intérêt de constituer une solution particulièrement simple de conception et de prix abordable, de sorte à pouvoir être proposée facilement à un patient, notamment pour un suivi à domicile dans le cadre par exemple d'une convalescence, d'une maladie chronique ou d'un essai clinique.

De plus, le dispositif d'analyse peut être changé à tout moment, tout en conservant le même dispositif de lecture. Cela évite les problèmes de calibrage rencontrés avec les dispositifs d'évaluation actuels.

Cette solution technique selon l'invention a encore l'intérêt de permettre une analyse des images numériques de manière centralisée (par exemple chez le promoteur et/ou l'investigateur d'un essai clinique) ce qui améliore la sécurité et la fiabilité des résultats.

Selon un mode de réalisation particulier, le système d'évaluation comprend plusieurs dispositifs de lecture associés à un dispositif d'analyse destiné à recevoir et à analyser les images numériques provenant de l'ensemble de ces dispositifs de lecture.

De préférence, les moyens de transmission comprennent un dispositif de transmission comprenant :
- des moyens de communication qui sont adaptés à l'échange de données avec ledit dispositif de lecture, notamment pour la transmission de ladite au moins une image numérique depuis ledit dispositif de lecture jusqu'audit dispositif de transmission,
- des moyens de télécommunication, pour la connexion et l'échange de données via le réseau de télécommunication.

Et les moyens de communication du dispositif d'analyse se présentent sous la forme de moyens de télécommunication, pour la connexion et l'échange de données via le réseau de télécommunication.

Dans ce cas, avantageusement, le dispositif de transmission comporte un écran, et les moyens de fourniture comprennent des moyens pour l'affichage du résultat de ladite évaluation qualitative et/ou quantitative sur ledit écran.

Encore dans ce cas, avantageusement, les moyens de communication équipant le dispositif de lecture et le dispositif de transmission consistent en des moyens de connexion sans fils et courte distance Bluetooth ou Wifi.

Toujours dans ce cas, avantageusement, le dispositif de transmission consiste en un téléphone mobile, en une tablette numérique ou en un ordinateur.

Selon d'autres caractéristiques avantageuses de réalisation :
- le dispositif de lecture est dépourvu de moyens de traitement de ladite au moins une image numérique, adaptés à l'évaluation qualitative et/ou quantitative dudit au moins un analyte susceptible d'être contenu dans ledit échantillon liquide, et éventuellement de moyens pour la fourniture du résultat d'évaluation qualitative et/ou quantitative ;
- le dispositif d'analyse comprend un programme d'ordinateur comprenant : des moyens de code de programme pour la réception de ladite au moins une image numérique depuis ledit dispositif de lecture, des moyens de code de programme pour traiter ladite au moins une image numérique, tenant compte de données de calibrage, des moyens de code de programme pour la fourniture du résultat de ladite évaluation qualitative et/ou quantitative, lorsque ledit programme d'ordinateur est exécuté par ledit dispositif d'analyse ;
- les moyens de réception du dispositif de lecture sont amovibles et interchangeables, de sorte à adapter lesdits moyens de réception en fonction du dispositif immunochromatographique à analyser ;
- le dispositif de lecture comporte des moyens pour la lecture de moyens d'identification portés par le dispositif immunochromatographique ;
- le système d'évaluation comprend encore un dispositif d'affichage distant, pour la fourniture du résultat d'évaluation qualitative et/ou quantitative à un tiers distant, lequel dispositif d'affichage distant est connectés au dispositif d'analyse par le biais du réseau de télécommunication, avantageusement Internet ; les moyens de fourniture du dispositif d'analyse comportent des moyens pour la transmission du résultat d'évaluation audit dispositif d'affichage distant via ledit réseau de télécommunication ;
- le système d'évaluation comprend un dispositif immunochromatographique comprenant : une zone amont de libération qui comprend au moins un réactif de détection conjugué avec un marqueur visible et/ou mesurable, ledit réactif de détection étant apte à se déplacer en conséquence de la migration de l'échantillon liquide dans ledit moyen de diffusion capillaire, et ladite zone aval de révélation qui comprend au moins un réactif de capture, immobilisé sur ledit moyen de diffusion capillaire ;
- le système d'évaluation comprend encore un dispositif de calibrage, destiné à être rapporté au niveau des moyens de réception et comportant des données de calibrage destinées à être lues préalablement au dispositif immunochromatographique à analyser, et le dispositif de lecture comporte des moyens pour l'acquisition des données de calibrage portées par ledit dispositif de calibrage ;
- le dispositif de lecture comporte encore des moyens pour le raccordement de moyens de communication filaire.

La présente invention concerne également le dispositif de lecture constitutif d'un système d'évaluation selon l'invention.

La présente invention concerne également le procédé pour l'évaluation qualitative et/ou quantitative d'au moins un analyte susceptible d'être contenu dans un échantillon liquide déposé sur un dispositif immunochromatographique, au moyen d'un système d'évaluation selon l'invention.

Ce procédé comprend les étapes successives suivantes :
- le cas échéant un appariement dudit dispositif de lecture avec ledit dispositif de transmission,
- une mise en place dudit dispositif immunochromatographique au niveau des moyens de réception du dispositif de lecture,
- une acquisition de ladite au moins une image numérique de la zone de révélation dudit dispositif immunochromatographique rapporté dans lesdits moyens de réception,
- une transmission de ladite au moins une image numérique depuis le dispositif de lecture jusqu'au dispositif de transmission, par le biais des moyens de communication,
- une transmission de ladite au moins une image numérique depuis ledit dispositif de transmission jusqu'audit dispositif d'analyse, via ledit réseau de télécommunication,
- un traitement de ladite au moins une image numérique par ledit dispositif d'analyse, pour l'évaluation qualitative et/ou quantitative dudit au moins un analyte susceptible d'être contenu dans ledit échantillon liquide, et
- une fourniture du résultat de ladite évaluation qualitative et/ou quantitative.

### DESCRIPTION DETAILLEE DE L'INVENTION

La présente invention sera encore illustrée, sans aucunement être limitée, par la description suivante en relation avec les figures annexées dans lesquelles :
- la figure 1 est une vue générale et schématique du système d'évaluation selon l'invention, et
- la figure 2 est une vue schématique d'un dispositif immunochromatographique constitutif dudit système d'évaluation.

Le système d'évaluation 1 selon l'invention est adapté à l'évaluation (ou détermination) qualitative et/ou quantitative d'au moins un analyte susceptible d'être contenu dans un échantillon liquide déposé sur un dispositif immunochromatographique 2.

Le système selon l'invention est intéressant en ce qu'il peut être adapté aussi bien à l'évaluation (ou détermination) de la présence d'au moins un analyte, qu'à l'évaluation (ou la détermination) de sa quantité.

Par « évaluer », « détecter » ou « déterminer », on entend ainsi la détermination qualitative (avantageusement la présence ou l'absence) d'un ou plusieurs analytes dans un échantillon liquide.

Par « évaluer », « détecter » ou « déterminer », on entend aussi la mesure et la quantification d'un ou plusieurs analytes dans un échantillon.

En effet, les performances du système d'évaluation selon l'invention autorisent également des modes de réalisation pour la réalisation de mesures quantitatives ou semiquantitatives d'un analyte ou d'au moins deux analytes différents dans un échantillon liquide.

Pour cela, le système d'évaluation 1 selon l'invention comprend ici :
- le dispositif immunochromatographique 2, sur lequel est destiné à être déposé un échantillon liquide d'intérêt,
- un dispositif de lecture 3, pour l'acquisition d'au moins une image numérique d'une zone de révélation de ce dispositif immunochromatographique 2,
- un dispositif de transmission 4, pour la transmission de ladite au moins une image numérique provenant dudit dispositif de lecture 3 jusqu'à un dispositif d'analyse 5 distant, et
- un dispositif d'analyse distant 4, pour le traitement de ladite au moins une image numérique provenant dudit dispositif de lecture 3 (via le dispositif de transmission 4) et pour la fourniture du résultat de l'évaluation qualitative et/ou quantitative dudit au moins un analyte susceptible d'être contenu dans l'échantillon liquide.

Selon l'invention, le dispositif de lecture 3, le dispositif de transmission 4 et le dispositif d'analyse distant 5 sont distincts structurellement les uns des autres, et sont destinées à être connectés par des moyens de communication pour l'échange de données en eux.

Par « distincts structurellement », on entend encore des dispositifs qui peuvent être écartés l'un de l'autre et qui sont mobiles indépendamment l'un de l'autre.

De manière alternative, non représentée, le système selon l'invention pourrait comporter d'autres moyens techniques pour assurer la télétransmission de ladite au moins une image numérique.

Les différents aspects de la présente invention sont présentés plus en détails ci-dessous.

### Analvte(s) et échantillon liquide

Par « analyte », on entend toute entité chimique, biochimique, ou biologique, que l'on souhaite détecter dans un échantillon.

Cette entité chimique consiste avantageusement en une entité issue du monde vivant, de préférence du monde végétal ou du monde animal, de préférence encore présent chez l'être humain.

Parmi les analytes détectés par les dispositifs et les procédés selon la présente invention, on citera notamment les protéines, les peptides, les anticorps, les hormones, les stéroïdes, les antigènes dérivés d'agents infectieux ou de cellules tumorales, les agents infectieux tels que les bactéries, les virus ou les parasites, les acides nucléiques (ADN ou ARN), les composés thérapeutiques, les drogues ou encore les antibiotiques.

L'analyte est en particulier choisi parmi ceux connus pour générer un effet crochet lors de leur détection par technique immunochromatographique.

Par « effet crochet » (ou « Hook effect » en anglais), on entend en particulier un résultat faux négatif obtenu par technique immunochromatographique, concluant de façon aberrante à l'absence de l'analyte dans l'échantillon, survenant lorsque l'analyte est présent dans l'échantillon à une concentration très élevée.

Généralement l'effet crochet produit un affaiblissement du signal réponse, visible et mesurable, inverse à l'augmentation de la concentration en analyte à déterminer, conduisant (i) à un signal identique (de même intensité) pour deux concentrations différentes, l'une faible et l'autre forte, et (ii) une inhibition de la révélation du signal pour une concentration en analyte extrêmement forte.

A cet égard, l'analyte est ainsi choisi avantageusement parmi l'hormone chorionique gonadotrope (ou « hCG »), l'antigène prostatique spécifique (PSA ou « Prostate-Specific Antigen »), l'hémoglobine, le FOB (« Fecal occult blood »), les marqueurs oncogéniques (tels que ferritine, AFP (alfa feto-protein), CA15-3/CA27.29 (cancer du sein), CA19-9 (cancer pancréatique), CA-125 (cancer ovarien)), la protéine C-réactive (« CRP » ou «C Reactive Protein »), la troponine I (« TNI » ou « Troponin I »), des marqueurs cardiaques (tels que troponine T, CK-MB, myoglobine, B-type natriuretic Peptide (t-BNP)), les DOA (pour « drugs of abuse »), les biomarqueurs pour le monitorage de thérapie (« Therapy monitoring biomarkers ») (TnF-alfa (tumor necrosis factor), autres thérapies associées avec des biomarqueurs oncogéniques circulants, autres biomarqueurs cellulaires, intracellulaires ou tissues spécifiques, etc.), l'hormone lutéinisante (« LH ») ou l'hormone folliculostimulante (« FSH »).

En fonction de leur structure, les dispositifs immunochromatographiques permettent la détermination d'un analyte unique (mono-analyte) ou la détermination de plusieurs analytes (poly-, ou multi- ou pluri-analytes).

Par « plusieurs analytes », on entend au moins deux analytes, de préférence encore 2, 3, 4 ou 5 analytes.

Cette approche multi-analytes peut être intéressante pour l'étude des maladies autoimmunes (« Auto immune disease panel »), pour l'étude des allergies (« Allergies panel »), pour le monitorage des thérapies (« therapy monitoring ») dans le domaine du médicaments, de la toxicologie, de la réponse du patient à un traitement ou des marqueurs inflammatoires, pour les tests de diagnostic d'infection multiple (par exemple virus de l'immunodéficience humaine ou VIH/hépatite C/hépatite B).

Par exemple, le dispositif immunochromatographique peut être adapté pour la détermination de différents anticorps dans un même échantillon liquide, à savoir par exemple anti-VIH (virus de l'immunodéficience humaine ou HIV), anti-HCV (virus de l'hépatite C), anti-HBs (marqueur de l'hépatite virale chronique), anti-TB (tuberculose).

Par « échantillon liquide », on entend tout échantillon dans lequel l'analyte recherché est en solution ou en suspension.

Cet échantillon liquide peut notamment être tout fluide biologique ou corporel.

L'échantillon liquide peut également avoir été obtenu directement ou indirectement à partir d'un fluide biologique ou corporel.

L'échantillon peut également être un extrait liquide d'un échantillon solide.

Typiquement, l'échantillon liquide est de l'urine, du sang total, du plasma ou du sérum.

### Dispositif immunochromatoaraphiaue

Le dispositif immunochromatographique 2 peut être choisi parmi les dispositifs immunochromatographiques existants ou développés à façon.

De tels dispositifs immunochromatographiques sont par exemple décrits dans le document WO-2013140089.

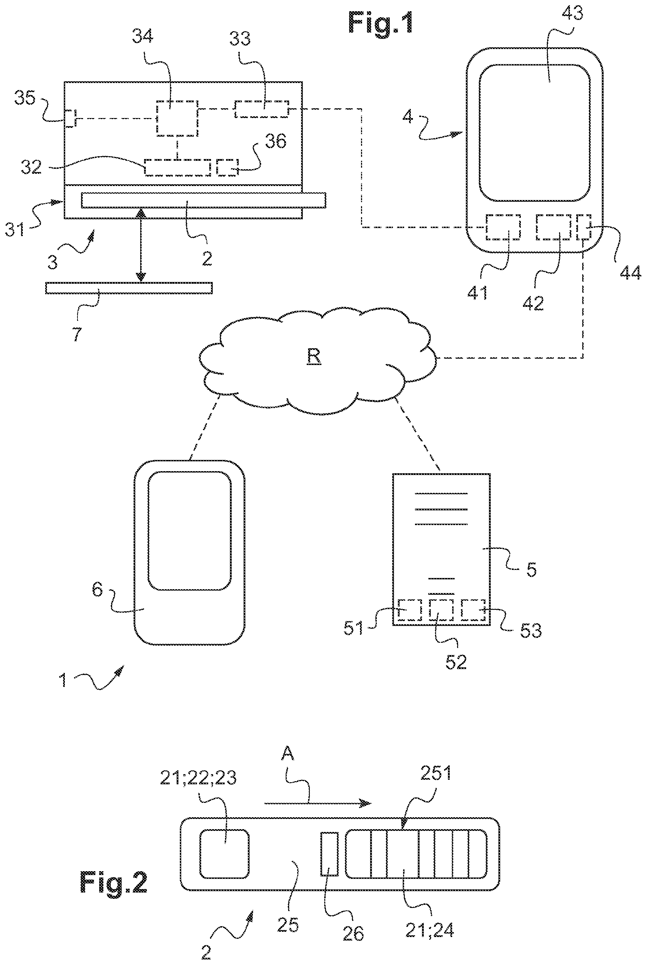

Tel que représenté sur la figure 2, le dispositif immunochromatographique 2 comporte habituellement un moyen de diffusion capillaire 21 sur lequel ledit échantillon liquide (non représenté) est destiné être déposé puis à migrer latéralement selon une direction et un sens de migration capillaire amont vers aval.

A titre d'exemple, ces moyens de diffusion capillaire 21 peuvent être constitués de divers supports immunochromatographiques, par exemple de cellulose, de nylon, de nitrocellulose, de polyéthylène ou de fibre de verre.

La direction et le sens de migration sont illustrés schématiquement par la flèche désignée par le repère A sur la figure 2.

De manière générale, les notions de « amont » et « aval » se réfèrent à cette direction et à ce sens de migration de l'échantillon liquide sur la longueur du moyen de diffusion capillaire 21.

Différentes zones successives sont matérialisées sur ce moyen de diffusion capillaire 21, dans ledit sens de migration capillaire amont vers aval A, à savoir au moins :
- une zone amont de dépôt 22, pour le dépôt de l'échantillon liquide,
- une zone amont de libération 23 qui comprend au moins un réactif de détection conjugué avec un marqueur visible et/ou mesurable, ledit réactif de détection étant apte à se déplacer en conséquence de la migration de l'échantillon liquide dans ledit moyen de diffusion capillaire 21, et
- une zone aval de révélation 24, qui comprend au moins un réactif de capture immobilisé sur ledit moyen de diffusion capillaire 21.

Dans des modes de réalisation particuliers, le dispositif 2 peut comporter au moins une zone supplémentaire de libération, qui est matérialisée sur ledit moyen de diffusion capillaire 21 et qui se situe en aval de l'un au moins des réactifs de capture, comme décrit dans le document WO-2013140089.

Le réactif de détection et/ou le réactif de capture sont choisis pour se lier spécifiquement avec ledit analyte et/ou à se lier spécifiquement l'un avec l'autre.

Cette approche assure la formation de complexe(s) permettant la détermination dudit analyte dans ledit échantillon liquide au niveau de la zone de révélation 24.

Les réactifs de détection et de capture sont en particulier choisis pour la mise en œuvre de tests immunologiques au format sandwich et/ou au format compétition.

En pratique, le dispositif immunochromatographique 2 peut par exemple être constitué d'un moyen de diffusion capillaire 21 formé par une bandelette chromatographique qui est fixée sur un support rigide 25.

Le support rigide 25 peut être constitué de matériaux divers tels que du carton, du carton plastifié ou plus préférentiellement de matières plastiques. De préférence, le support rigide est constitué de polystyrène.

Avantageusement, le moyen de diffusion capillaire 21 peut être incorporé dans le support rigide 25.

Ce support rigide 25 facilite alors la manipulation du moyen de diffusion capillaire 21, et peut également protéger celui-ci notamment de l'humidité.

Le support rigide 25 peut envelopper partiellement ou totalement le moyen de diffusion capillaire 21.

Le support rigide 25 peut être constitué de matériaux divers tels que du carton, du carton plastifié ou plus préférentiellement de matières plastiques. De façon avantageuse, le support de préhension est constitué d'un matériau rigide et imperméable.

Ces supports rigides 25 ou boîtiers sont notamment décrits dans les brevets WO-2007/023372, EP-0 291 194, EP-0 560 411, EP-0 560 410 et EP-1 091 808.

Habituellement, le support rigide 25 est en forme de boîtier.

Ce support rigide 25 est avantageusement pourvu d'au moins une fenêtre d'observation 251 permettant d'observer la zone de révélation 24.

Ces fenêtres d'observation 251 sont avantageusement agencées de sorte à offrir un accès visuel direct uniquement à la zone de révélation à analyser pour la détermination d'un analyte, ou le cas échéant pour la détermination d'au moins deux analytes.

Le dispositif immunochromatographique 2 peut encore embarquer des moyens d'identification 26, qui sont portés par exemple par son support rigide 25.

Ces moyens d'identification 26 contiennent en particulier un numéro d'identification unique pour le suivi de ce dispositif immunochromatographique 2 et pour une gestion optimale des résultats.

Ces moyens d'identification 26 consistent avantageusement en des moyens d'identification optique, par exemple du type code-barres ou codes 2D (QR codes).

De manière alternative, les moyens d'identification 26 sont du type communication en champ proche ou NFC.

Ces moyens d'identification 26 consistent avantageusement en une puce NFC.

Le dispositif immunochromatographique 2 peut encore embarquer des moyens visuels pour assister le recadrage de l'image lors de l'analyse.

Les moyens de recadrage consistent par exemple en au moins deux points fixes, avantageusement portés directement par le support rigide 25, de sorte à définir un axe longitudinal dudit dispositif immunochromatographique 2.

Ces moyens de recadrage permettent de tenir compte d'éventuelles variations entre les différents dispositifs de lecture et ainsi d'améliorer la reproductibilité des résultats.

### Réactif de détection et réactif de capture

Sur sa longueur, le moyen de diffusion capillaire 21 comporte, d'une part, au moins un réactif de détection réparti pour matérialiser des parties de libération et, d'autre part, au moins un réactif de capture réparti pour matérialiser des parties de capture.

Par « partie de libération » ou « partie de capture » décrites plus en détails par la suite, on entend une partie localisée et délimitée du moyen de diffusion capillaire 21 sur lequel a été déposé une quantité d'au moins un réactif de détection ou d'au moins un réactif de capture, respectivement.

Chacune des parties de libération et parties de capture consiste avantageusement en une ligne ou bande transversale (s'étendant perpendiculairement à la direction de migration), présentant par exemple une largeur comprise entre 1 et 2 mm, et une surface comprise entre 3 et 5 mm².

De manière générale, le « réactif de détection » ou le « réactif de capture » consiste en toute entité chimique, biochimique ou biologique, qui est apte à se lier spécifiquement pour former un complexe permettant la détermination dudit analyte dans l'échantillon liquide.

Le réactif de détection et/ou le réactif de capture constituent encore des réactifs dits de « liaison ».

De tels réactifs de liaison, permettant la détermination de l'analyte ou de plusieurs analytes dans l'échantillon liquide, sont bien connus et peuvent être choisis à façon pour la mise en œuvre de l'invention.

Ces réactifs de liaison sont choisis avantageusement parmi ceux qui sont aptes à se lier spécifiquement avec ledit analyte et/ou à se lier spécifiquement l'un avec l'autre.

Selon le format de test mis en œuvre, les réactifs de liaison complémentaires sont destinés à former des complexes différents :
- les réactifs de liaison sont aptes à se fixer concomitamment à l'analyte, pour former un test au format sandwich,
- l'un des réactifs de liaison (détection ou capture) est apte à se fixer à l'analyte mais aussi à l'autre réactif de liaison (respectivement capture ou détection), pour former un test au format compétition.

Dans ce cadre, l'un au moins des réactifs de liaison est avantageusement choisi parmi les entités chimiques, biochimiques ou biologiques, aptes à se lier spécifiquement avec l'analyte et/ou à se lier spécifiquement avec un analogue de l'analyte.

Par « lier » ou « liaison », on entend toute liaison forte, par exemple covalente, mais aussi toute liaison faible, par exemple du type antigène/anticorps ou analyte / anti-analyte.

Par « anti-analyte », on entend toute entité chimique, biochimique, ou biologique, susceptible de se lier spécifiquement avec l'analyte, ou avec le réactif de capture en compétition avec l'analyte, par exemple un anticorps, un antigène ou un acide nucléique.

Par « analogue approprié de l'analyte », on entend avantageusement toute entité chimique, biochimique ou biologique, apte à se lier de manière spécifique au réactif de capture ou au réactif de détection, selon le cas, en compétition avec l'analyte.

Les réactifs de liaison sont avantageusement choisis parmi les anticorps, les antigènes ou les acides nucléiques.

L'analyte et le réactif de liaison forment ainsi typiquement un couple apte à se lier spécifiquement l'un avec l'autre, comme par exemple un couple ligand/anti-ligand, un couple antigène/anticorps, un couple ADN/ARN ou un couple ADN/ADN.

Ainsi, si l'analyte est un antigène ou un haptène, l'un au moins des réactifs de liaison (le réactif de détection et/ou le réactif de capture) est avantageusement un anticorps spécifique de l'analyte.

Par « anticorps spécifique de l'analyte », on entend un anticorps capable de se lier spécifiquement avec l'analyte dans une liaison de type antigène/anticorps.

Il s'agit typiquement d'un anticorps polyclonal ou d'un anticorps monoclonal, ayant une forte affinité pour l'analyte. De préférence, il s'agit d'un anticorps monoclonal.

Si l'analyte est un anticorps, l'un au moins des réactifs de liaison est avantageusement l'antigène reconnu par l'anticorps.

Si l'analyte est un acide nucléique, l'un au moins des réactifs de liaison est avantageusement une sonde ADN complémentaire.

Le ou les réactifs de détection sont avantageusement conjugués à un marqueur visible et/ou mesurable, avantageusement un marqueur particulaire.

Par « marqueur visible et/ou mesurable », on entend tout marquage permettant une détection directe ou indirecte à l'œil nu, ou à l'aide de l'appareil de lecture, en raison de l'émission d'un signal au niveau de la zone de révélation.

Le signal est par exemple une fluorescence ou une coloration.

On citera par exemple les marqueurs particulaires colorés comme l'or colloïdal, ou fluorescents, les particules de latex colorées, les particules de latex fluorescentes et les particules conjuguées à l'avidine et à la streptavidine.

Les marqueurs particulaires, colorés ou fluorescents, consistent ainsi en des particules de petite taille insolubles dans l'eau et qui forment donc des suspensions, dispersions ou solutions, en phase liquide.

Parmi les marqueurs permettant une observation directe à l'œil nu, on citera aussi les marqueurs de type dextran (Hansen T.M., IVD Technology 4, 35-40, 2003). Le réactif de liaison est alors conjugué à une chaîne de dextran (dérivé de polysaccharide) portant des fluorophores.

Les marqueurs peuvent également consister en des enzymes (la phosphatase alcaline ou AP, la peroxydase de raifort ou HRP, notamment), en des colorants (ou « dyes ») ou en des composés chimiluminescents (notamment l'isothiocyanate de fluorescéine ou FITC).

Pour augmenter la sensibilité, on peut avoir recours par exemple, à un anticorps marqué selon des techniques connues de l'homme de l'art pour une détection indirecte, comme par exemple un anticorps biotinylé, permettant indirectement une détection par la formation des entités avidine-biotine et streptavidine-biotine.

Cet anticorps marqué et biotinylé peut également, soit être directement déjà déposé sur une ligne-test, dans la zone de capture, pour augmenter la sensibilité, soit être déposé avec l'anticorps de détection spécifique, pour augmenter le temps de contact et encore la sensibilité notamment, par exemple, en raison du nombre de sites de fixation.

De son côté, le réactif de capture spécifique de l'analyte est immobilisé sur le support solide selon des techniques connues de l'homme du métier.

Ce réactif de capture est immobilisé de telle façon qu'il ne soit pas mobile à l'état humide.

Cette immobilisation peut s'effectuer par exemple par absorption ou par un couplage covalent.

### Dispositif de lecture

Le dispositif de lecture 3 du système d'évaluation 1 consiste avantageusement en un appareil portatif.

Ce dispositif de lecture 3 selon l'invention, avantageusement en forme d'un boîtier et désigné encore sous l'appellation de « lecteur », comprend en particulier :
- des moyens 31 pour la réception du dispositif immunochromatographique 2,
- des moyens 32 pour l'acquisition d'au moins une image numérique de la zone de révélation 24 du dispositif immunochromatographique 2 rapporté dans les moyens de réception 31 précités, et
- des moyens de communication 33 qui sont adaptés à l'échange de données avec au moins le dispositif de transmission 4.

Ce dispositif de lecture 3 comporte encore avantageusement des moyens de commande 34, par exemple sous la forme d'un microcontrôleur intégrant un programme d'ordinateur (firmware), pour le pilotage et la coordination de ses composants électroniques.

Les moyens de réception 31 comportent un emplacement pour le positionnement et le maintien du dispositif immunochromatographique 2, par exemple par emboîtement amovible.

Les moyens de réception 31 consistent avantageusement en une partie amovible et interchangeable du dispositif de lecture 3.

Cette particularité structurelle permet d'adapter ces moyens de réception 31, parmi une gamme à disposition, en fonction du dispositif immunochromatographique 2 à analyser (en particulier pour tenir compte de la forme de son support rigide 25).

Pour cela, les moyens de réception 31 comportent des moyens de solidarisation (non représentés) qui permettent une solidarisation démontable avec une partie complémentaire du dispositif de lecture 3 (intégrant notamment les composants électroniques précités).

Ces moyens de solidarisation amovible consistent par exemple en des structures d'emboîtement par déformation élastique (clipsage).

Les moyens de réception 31 permettent également un positionnement de la zone de révélation 24 du dispositif immunochromatographique 2 de manière optimale par rapport aux moyens d'acquisition 32.

Les moyens d'acquisition 32 comprennent avantageusement :
- un capteur optique (ou photographique),
- un convertisseur analogique-numérique, et
- un module de traitement, comprenant un processeur et un programme d'ordinateur qui sont avantageusement adaptés à générer une image numérique à partir des données provenant du convertisseur.

De tels moyens d'acquisition 32 sont connus en soi de l'homme du métier.

Le capteur optique est un composant électronique photosensible servant à convertir un rayonnement électromagnétique (UV, visible ou IR) en un signal électrique analogique.

Ce capteur optique est avantageusement choisi parmi :
- les capteurs CCD (pour « Charge-Coupled Device » ou « dispositif à transfert de charge » (DTC)) et
- les capteurs CMOS (pour « Complementarity metal-oxide-semiconductor »).

Le signal obtenu est numérisé par le convertisseur analogique-numérique, puis traité par le module de traitement pour obtenir une image numérique d'intérêt correspondant à la zone de révélation 24 du dispositif immunochromatographique 2.

De manière générale, par « image numérique », on entend une image constituée d'un ensemble de pixels, dont chacun est défini par plusieurs paramètres (par exemple la couleur et la luminosité).

Cette image numérique se présente avantageusement sous la forme d'un fichier image, c'est-à-dire un fichier informatique dont le contenu est constitué d'au moins une image.

Selon le type de codage utilisé, le fichier image consiste en :
- un fichier image en mode vectoriel, ou
- un fichier image en mode matriciel ou mode points (bitmap image).

Au sein du dispositif de lecture 3, l'image numérique se présente ainsi avantageusement dans un format natif ou « brut », par exemple du type fichier RAW.

Un tel fichier contient ainsi toutes les données enregistrées par le capteur optique.

De manière alternative, les données numériques issues du convertisseur sont directement transmises au dispositif d'analyse 4 pour son traitement et pour l'obtention de l'image numérique d'intérêt.

Avantageusement, le dispositif de lecture 3 est ainsi dépourvu :
- de moyens de traitement de ladite au moins une image numérique, adaptés à l'évaluation qualitative et/ou quantitative dudit au moins un analyte susceptible d'être contenu dans ledit échantillon liquide, et
- éventuellement de moyens pour la fourniture du résultat d'évaluation qualitative et/ou quantitative.

Les moyens d'acquisition 32 constituent ici également des moyens pour la lecture des moyens d'identification 26 portés par le dispositif immunochromatographique 2.

Dans ce cas, ces moyens d'identification 26 consistent en des moyens d'identification optique, par exemple du type code-barres ou codes 2D (QR codes).

De manière alternative, le dispositif de lecture 3 peut encore comporter des moyens de lecture (non représentés) adaptés à coopérer avec des moyens d'identification 26 du type communication en champ proche ou NFC, à savoir un lecteur NFC.

Les moyens de communication 33 comprennent quant à eux des moyens filaires ou des moyens sans fil, adaptés à l'échange de données avec au moins le dispositif de transmission 4, notamment pour la transmission de ladite au moins une image numérique depuis ledit dispositif de lecture 3 jusqu'audit dispositif de transmission 4.

Ces moyens de communication 33 consistent avantageusement en des moyens de connexion sans fils et courte distance, par exemple Bluetooth, Wifi ou NFC.

De manière alternative ou complémentaire, non représentée, les moyens de communication 33 peuvent comporter des moyens de télécommunication au travers d'un réseau de télécommunication R, par exemple Internet, adaptés à l'échange de données avec le dispositif d'analyse 5 à distance.

Les moyens de communication 33 forment alors une partie des moyens de transmission pour transmettre, via un réseau de télécommunication R, ladite au moins une image numérique acquise par ledit dispositif de lecture 3 jusqu'audit dispositif d'analyse 5 situé à distance.

Le dispositif de lecture 2 peut encore comporter l'un au moins des moyens choisis parmi les moyens suivants :
- des moyens 35 pour le raccordement de moyens de communication filaire,
- des moyens 36 pour l'éclairage de la zone de révélation 24 du dispositif immunochromatographique 2, et
- des moyens pour l'alimentation autonome en énergie électrique, par exemple une batterie électrique.

Les moyens de raccordement 35 consistent par exemple en une prise informatique ou bus.

Ces moyens de raccordement 35 sont en particulier utiles dans le cas d'un raccordement en vue d'une mise à jour du dispositif de lecture 3, notamment de ses moyens de commande 34.

Les moyens d'éclairage 36 consistent quant à eux en par exemple en un ensemble de diodes électroluminescentes ou LED, convenablement disposées pour l'éclairage de la zone de révélation 24 du dispositif immunochromatographique 2 lors de sa lecture.

### Dispositif de transmission

Le dispositif de transmission 4 consiste avantageusement en un téléphone mobile, ou en une tablette numérique ou en un ordinateur, distinct du dispositif de lecture 3.

Par « téléphone mobile », on entend en particulier un appareil téléphonique qui établit des liens de communication par ondes radio et qui est utilisable en se déplaçant.

Parmi ces téléphones mobiles, on entend en particulier un téléphone intelligent ou smartphone, qui est un téléphone cellulaire qui, en plus d'offrir des fonctions téléphoniques, intègre un assistant numérique personnel qui le transforme en un outil de communication hybride capable de traiter et de transmettre des données informatiques ou multimédias par voie radioélectrique.

Par « tablette numérique », on entend un appareil portatif en forme de tablette, dépourvu de clavier, ayant pour seule interface un écran tactile, qui offre de nombreuses possibilités de personnalisation, intègre plusieurs applications et permet l'accès à un réseau (par exemple un réseau de télécommunication, notamment Internet).

Le dispositif de transmission 4 sert de moyens de connexion informatique (ou passerelle) entre le dispositif de lecture 2 et le dispositif d'analyse 5, notamment pour la transmission de ladite au moins une image numérique acquise depuis le dispositif de lecture 2 jusqu'au dispositif d'analyse 5.

Le dispositif de transmission 4 comprend :
- des moyens de communication 41 qui sont adaptés à l'échange de données avec au moins avec le dispositif de lecture 3,
- éventuellement des moyens 42 pour le traitement de ladite au moins une image numérique provenant du dispositif de lecture 3,
- un écran 43, servant d'interface pour l'utilisateur (périphérique de sortie destiné à la représentation visuelle temporaire des données), et
- des moyens de télécommunication 44, pour la connexion et l'échange de données via un réseau de télécommunication R, avantageusement Internet.

Les moyens de communication 41 sont adaptés notamment pour la réception de ladite au moins une image numérique depuis le dispositif de lecture 3, et avantageusement pour le pilotage de ce dispositif de lecture 3.

Là encore, ces moyens de communication 33 consistent avantageusement en des moyens de connexion sans fils et courte distance, par exemple Bluetooth ou Wifi ou NFC.

Les moyens de traitement 42, optionnels, sont adaptés à l'évaluation qualitative et/ou quantitative dudit au moins un analyte susceptible d'être contenu dans ledit échantillon liquide.

Pour cela, ces moyens de traitement 42 font partie de moyens de commande qui comprennent avantageusement des moyens de calcul (par exemple au moins un processeur ou CPU) associés à un programme d'ordinateur enregistré sur des moyens de mémoire.

Le programme d'ordinateur comprend avantageusement :
- des moyens de code de programme pour la réception de ladite au moins une image numérique depuis ledit dispositif de lecture 3,
- des moyens de code de programme pour la transmission de ladite au moins une image numérique jusqu'audit dispositif d'analyse 5, via les moyens de télécommunication 44, et
- éventuellement des moyens de code de programme pour traiter ladite au moins une image numérique (par exemple sous la forme d'un algorithme développé à façon), tenant compte de données de calibrage pour obtenir un résultat d'une évaluation qualitative et/ou quantitative (formant ici les moyens de traitement proprement dit),
- le cas échéant des moyens de code de programme pour la fourniture du résultat de ladite évaluation qualitative et/ou quantitative (formant des moyens de fourniture), qui consistent avantageusement en des moyens pour l'affichage du résultat de ladite évaluation sur l'écran 43,
lorsque ledit programme d'ordinateur est exécuté par ledit dispositif de transmission 4.

Ce programme d'ordinateur consiste avantageusement en une application mobile, c'est-à-dire un logiciel applicatif développé pour être installé sur un appareil électronique mobile (par exemple téléphone mobile ou tablette).

Une telle application peut être installée sur l'appareil dès la conception de celui-ci ou bien, si l'appareil le permet, téléchargée par l'utilisateur par le biais d'une boutique en ligne.

Les moyens de télécommunication 44 consistent avantageusement en des moyens pour l'échange de données, par exemple s'appuyant sur les normes de téléphonie mobile : Universal Mobile Telecommunications System (UMTS) ou Long Term Evolution (LTE).

Plus généralement, l'accès au réseau de télécommunication R avec le dispositif de transmission 4 peut être obtenu grâce à un fournisseur d'accès via divers moyens de communication électronique : filaire (réseau téléphonique commuté (bas débit), ADSL, fibre optique), ou sans fil (WiMAX, par satellite, 4G).

Ces moyens de télécommunication 44 sont adaptés notamment pour la transmission de ladite au moins une image numérique, voire de résultat de ladite évaluation qualitative et/ou quantitative, jusqu'au dispositif d'analyse 5.

### Dispositif d'analyse à distance

L'image numérique acquise par le dispositif de lecture 3 est transmise, via le réseau de télécommunication R, jusqu'au dispositif d'analyse 5 situé à distance (par exemple un serveur informatique dédié).

En pratique, le dispositif d'analyse 5, distant, est destiné à recevoir des données depuis une pluralité de dispositifs de transmission 4.

Le résultat d'évaluation peut alors être obtenu et fourni par ce dispositif d'analyse 5, à façon et via le réseau de télécommunication R, jusqu'à au moins un dispositif « récepteur » distant (par exemple en possession du patient et/ou du médecin traitant).

Le dispositif d'analyse distant 5 comprend :
- des moyens 51 pour le traitement de ladite au moins une image numérique provenant du dispositif de lecture 3, via le dispositif de transmission 4,
- des moyens de télécommunication 52, pour la connexion et l'échange de données via le réseau de télécommunication R, avantageusement Internet,
- des moyens mémoires 53 pour la collecte des données dans une base de données.

Les moyens de télécommunication 52 sont adaptés notamment pour la réception de ladite au moins une image numérique (voire de résultat d'évaluation qualitative et/ou quantitative) depuis le dispositif de transmission 4.

Ces moyens de télécommunication 52 consistent avantageusement en des moyens pour l'échange de données, par exemple s'appuyant sur les normes de téléphonie mobile : Universal Mobile Telecommunications System (UMTS) ou Long Term Evolution (LTE).

Plus généralement, l'accès au réseau de télécommunication R avec le dispositif d'analyse 5 peut être obtenu grâce à un fournisseur d'accès via divers moyens de communication électronique : filaire (réseau téléphonique commuté (bas débit), ADSL, fibre optique), ou sans fil (WiMAX, par satellite, 4G).

Les moyens de traitement 51 sont adaptés à l'évaluation qualitative et/ou quantitative dudit au moins un analyte susceptible d'être contenu dans ledit échantillon liquide.

Pour cela, ces moyens de traitement 51 font partie de moyens de commande qui comprennent avantageusement des moyens de calcul (par exemple au moins un processeur ou CPU) associés à un programme d'ordinateur enregistré sur des moyens de mémoire.

Le programme d'ordinateur comprend avantageusement :
- des moyens de code de programme pour la réception de ladite au moins une image numérique depuis ledit dispositif de transmission 4 (voire directement depuis le dispositif de lecture 3, sans passer par le dispositif de transmission 4), via les moyens de télécommunication 52, et
- des moyens de code de programme pour traiter ladite au moins une image numérique (par exemple sous la forme d'un algorithme développé à façon), tenant compte de données de calibrage pour obtenir un résultat d'une évaluation qualitative et/ou quantitative (formant ici les moyens de traitement proprement dit),
- des moyens de code de programme pour la fourniture et la transmission du résultat de ladite évaluation qualitative et/ou quantitative à au moins un dispositif d'affichage (formant des moyens de fourniture), avantageusement via les moyens de télécommunication 52,
- et éventuellement des moyens pour effectuer une comparaison entre, d'une part, le résultat de l'évaluation qualitative et/ou quantitative issu du dispositif d'analyse 5 et, d'autre part, le résultat de l'évaluation qualitative et/ou quantitative issu du dispositif de transmission 4,
lorsque ledit programme d'ordinateur est exécuté par ledit dispositif d'analyse distant 5.

Les moyens de code de programme pour la fourniture et la transmission du résultat peuvent encore se présenter sous la forme d'un portail d'accès Web, auquel les personnes autorisées peuvent se connecter pour obtenir les résultats issus des analyses.

### Autres éléments du système d'évaluation

Le système d'évaluation peut encore comprendre un dispositif d'affichage distant 6, pour la fourniture du résultat d'évaluation qualitative et/ou quantitative à un tiers distant, par exemple à un médecin traitant.

Pour cela, le serveur distant 5 et le dispositif d'affichage distant 6 sont connectés par le biais du réseau de télécommunication R, avantageusement Internet.

Les moyens de fourniture du dispositif d'analyse distant 5 comportent alors avantageusement des moyens pour la transmission du résultat d'évaluation audit dispositif de transmission 4 et/ou audit dispositif d'affichage distant 6, via ledit réseau de télécommunication R, par le biais de ses moyens de télécommunication 52.

Le système d'évaluation 1 peut encore comprendre un dispositif de calibrage 7, destiné à être rapporté au niveau des moyens de réception 31 du dispositif de lecture 3, à l'emplacement habituel du dispositif immunochromatographique 2 (figure 1).

Le dispositif de calibrage 7 a une forme similaire au dispositif immunochromatographique 2 à analyser.

Ce dispositif de calibrage 7 comporte des données de calibrage destinées à être acquises préalablement à une lecture d'un dispositif immunochromatographique 2 à analyser.

Ces données de calibrage contiennent des données utiles à l'interprétation du résultat de la zone de révélation 24 du dispositif immunochromatographique 2, par exemple :
- des algorithmes dédiés, et
- des courbes « dose-réponse » (concentration-signal).

Dans ce cas, le dispositif de lecture 3 comporte avantageusement des moyens pour l'acquisition de ces données de calibrage portées par ledit dispositif de calibrage 7.

Ces données de calibrage se présentent ici pour cela sous forme optique, par exemple du type code-barres ou codes 2D (QR codes).

Ce dispositif de calibrage 7 peut encore comporter des lignes comportant des intensités différentes, qui sont fonction des seuils recherchés, de manière à neutraliser les différences entre les lots.

De manière alternative, le dispositif de lecture 3 peut encore comporter des moyens de lecture (lecteur) adaptés à coopérer avec des moyens d'identification 26 (puce) sous une forme « communication en champ proche » ou NFC.

Ces données de calibrage sont ensuite transmises, ici via le dispositif de transmission 4, jusqu'au dispositif d'analyse 5 en vue de l'interprétation de dispositifs immunochromatographiques 2 associés à ces données.

### Procédé d'évaluation

Tout d'abord, le dispositif de lecture 3 est apparié avec ledit dispositif de transmission 4, de sorte à permettre un échange de données au travers de leurs moyens de communication 33, 41 respectifs.

Avant la lecture d'un dispositif immunochromatographique 2, il est possible de récupérer les données de calibrage sur un dispositif de calibrage 7 associé à ce dispositif immunochromatographique 2.

Pour cela, le dispositif de calibrage 7 est rapporté au niveau du dispositif de lecture 3, de sorte à collecter les données de calibrage qu'il comporte puis à les transmettre au dispositif de transmission 4.

Cette approche permet une utilisation du système 1 selon l'invention de manière autonome, sans nécessité une connexion au réseau Internet par exemple.

Ensuite, l'échantillon liquide à analyser est déposé dans la zone de dépôt 22 du dispositif immunochromatographique 2.

Ce dispositif immunochromatographique 2 peut alors être rapporté immédiatement, ou après un laps de temps déterminé, au niveau des moyens de réception 31 du dispositif de lecture 3.

On attend un temps suffisant pour la migration par diffusion capillaire de l'échantillon liquide jusqu'à la zone de révélation 24 du dispositif immunochromatographique 2.

Ce temps suffisant peut être suivi :
- manuellement, dans laquelle un individu déclenche l'acquisition de l'image numérique de la zone de révélation 24, ou
- automatiquement (tempérage), dans laquelle le dispositif de lecture 3 ou le dispositif d'analyse 4 déclenche de manière autonome l'acquisition de l'image numérique de la zone de révélation 24.

Dans ce second cas, les données de calibrage contiennent avantageusement une valeur optimale de ce laps de temps.

Le dispositif de lecture 3 effectue alors une acquisition d'une image numérique de la zone de révélation 24 du dispositif immunochromatographique 2 en présence.

Cette image numérique est ensuite transmise depuis le dispositif de lecture 3 « émetteur » jusqu'au dispositif de transmission 4 « récepteur », par le biais de leurs moyens de communication 33, 41.

L'image numérique est pour cela convertie ici en un train numérique entre ces deux moyens de communication 33, 41.

Cette image numérique est ensuite transmise (télétransmise) par le dispositif de transmission 4 « émetteur », immédiatement ou ultérieurement, jusqu'au dispositif d'analyse 5 « récepteur », par le biais de leurs moyens de télécommunication 44, 52.

La « transmission ultérieurement » permet une mise en attente de la transmission des données, par exemple dans le cas où les moyens de télécommunication 44 sont inactifs et en attente d'une connexion au dispositif d'analyse 5 (notamment lorsque le dispositif de transmission 4 n'est pas connecté à Internet ou lorsqu'il existe de problèmes de connexion entre le dispositif de transmission 4 et le dispositif d'analyse 5).

Cette transmission peut être effectuée automatiquement par un service fonctionnant en tâche de fond sur le dispositif de transmission 4.

En pratique, le dispositif d'analyse 5 est destiné à recevoir des images numériques en provenant d'une pluralité de dispositifs de lecture 3.

Chaque image numérique, reçue par le dispositif d'analyse 5, peut faire l'objet d'un traitement par le programme d'ordinateur des moyens de traitement 51 (algorithme), de manière à déterminer le profil de la zone de révélation 24.

Ce profil de la zone de révélation 24 peut être issu de deux phénomènes :
- le réactif de détection, complexé avec l'analyte, se fixe au réactif de capture complémentaire (format sandwich), et/ou
- le réactif de détection se fixe au réactif de capture (formant compétition).

La zone de révélation 24 comporte ainsi des complexes créant un profil (ou signal) visible et/ou mesurable, généralement des bandes parallèles entre elles et perpendiculaire au sens de migration A.

Ce profil est en particulier caractérisé par une intensité de signal, voire par le nombre et l'agencement des bandes et leurs intensités respectives.

Cette intensité du signal est par exemple exprimée en nombre de pixels, détecté sur tout ou partie de la zone de révélation 24.

Le profil de la zone de révélation 24, notamment son intensité, est alors comparé par rapport aux données de calibrage, cela par la mise en œuvre des moyens de traitement 51 du dispositif d'analyse 5 (en particulier les moyens de code de programme précités pour traiter ladite au moins une image numérique).

Les moyens de traitement 51 évaluent ainsi qualitativement et/ou semi-quantitativement et/ou quantitativement l'analyte ou les analytes à partir du profil obtenu.

Il est alors possible de transformer ce résultat « profil » en une valeur semi-quantitative, tenant compte des données de calibrage (notamment d'une courbe dose-réponse).

Le résultat visuel obtenu peut ainsi être transformé par exemple en une concentration, exprimée par exemple en ng par mL ou en mUI par mL de l'analyte dans l'échantillon liquide à analyser.

Le résultat d'évaluation est avantageusement enregistré sur des moyens mémoires 53 du dispositif d'analyse 5.

Une fourniture du résultat de ladite évaluation qualitative et/ou quantitative peut être mise en œuvre par le dispositif d'analyse 5 (en particulier par l'exécution du programme d'ordinateur des moyens de traitement), par transmission jusqu'à différentes destinations possibles, par exemple :
- l'écran 43 du dispositif de transmission 4, et/ou
- le dispositif d'affichage distant 6, pour la fourniture du résultat d'évaluation qualitative et/ou quantitative au tiers distant.

Ce résultat d'évaluation est avantageusement associé à des données de contexte (relatives par exemple au patient et au test mise en œuvre), notamment :
- type de dispositif immunochromatographique 2,
- identification du patient,
- horodatage,
- localisation du patient.

Le cas échéant, l'image numérique, reçue par le dispositif de transmission 4, peut faire l'objet d'un traitement identique ou similaire par le programme d'ordinateur de ses moyens de traitement 42 (algorithme), pour la fourniture directement du résultat de ladite évaluation qualitative et/ou quantitative sur l'écran 43.

Une telle analyse est en particulier utile lorsque le dispositif de transmission 4 n'est pas connecté au dispositif d'analyse 5.

Le résultat peut alors également être transmis au dispositif d'analyse 5, en accompagnement de l'image numérique correspondant à ce résultat.

Le dispositif d'analyse 5 peut éventuellement effectuer une comparaison entre, d'une part, le résultat de l'évaluation qualitative et/ou quantitative issu de ce dispositif d'analyse 5 et, d'autre part, le résultat de l'évaluation qualitative et/ou quantitative issu du dispositif de transmission 4.

Dans le cas où une différence est observée, une mise à jour du dispositif de transmission 4 est sollicitée.

De manière générale, la solution technique selon l'invention a notamment l'intérêt de permettre une analyse des images numériques de manière centralisée, ce qui améliore la sécurité et la fiabilité des résultats.

Cette solution est également intéressante dans le cas d'un essai clinique, notamment multicentrique, dans lequel le dispositif d'analyse 5 est centralisé par exemple chez le promoteur et/ou l'investigateur de manière à assurer une gestion et un suivi optimaux de cet essai (notamment chez une Société de Recherche sous Contrat dit encore « CRO » pour « Contract Research Organization » ou chez une industrie pharmaceutique).

## Revendications

1. Système pour l'évaluation qualitative et/ou quantitative d'au moins un analyte susceptible d'être contenu dans un échantillon liquide déposé sur un dispositif immunochromatographique (2),
lequel dispositif immunochromatographique (2) comporte des moyens de diffusion capillaire (21) sur lesquels ledit échantillon liquide est destiné à migrer depuis une zone amont de dépôt (22) jusqu'à une zone aval de révélation (24),
**caractérisé en ce que** ledit système d'évaluation (1) comprend au moins un dispositif de lecture (3), un dispositif de transmission (4) et au moins un dispositif d'analyse (5), qui sont distincts structurellement les uns des autres,
**en ce que** ledit au moins un dispositif de lecture (3), formant un appareil portatif, comporte :
- des moyens (31) pour la réception dudit dispositif immunochromatographique (2), comportant un emplacement pour le positionnement et le maintien du dispositif immunochromatographique (2),
- des moyens (32) pour l'acquisition d'au moins une image numérique de la zone de révélation (24) dudit dispositif immunochromatographique (2) rapporté dans lesdits moyens de réception (31), lesquels moyens d'acquisition (32) comprennent un capteur photographique,
- des moyens de communication (33) qui sont adaptés à l'échange de données avec au moins le dispositif de transmission (4), pour la transmission de ladite au moins une image numérique depuis ledit dispositif de lecture (3),
- des moyens (36) pour l'éclairage de la zone de révélation (24) du dispositif immunochromatographique (2),
- des moyens pour l'alimentation autonome en énergie électrique, comprenant une batterie électrique,
lequel au moins un dispositif de lecture (3) est dépourvu :
- de moyens de traitement de ladite au moins une image numérique, adaptés à l'évaluation qualitative et/ou quantitative dudit au moins un analyte susceptible d'être contenu dans ledit échantillon liquide, et
- de moyens pour la fourniture du résultat d'évaluation qualitative et/ou quantitative,
**en ce que** ledit au moins un dispositif d'analyse (5) comprend :
- des moyens de communication (52) qui sont adaptés à l'échange de données, pour la réception de ladite au moins une image numérique, lesquels moyens de communication (52) du dispositif d'analyse (5) se présentent sous la forme de moyens de télécommunication, pour la connexion et l'échange de données via le réseau de télécommunication (R),
- des moyens (51) pour le traitement de ladite au moins une image numérique, adaptés à l'évaluation qualitative et/ou quantitative dudit au moins un analyte susceptible d'être contenu dans ledit échantillon liquide, et
- des moyens pour la fourniture du résultat de ladite évaluation qualitative et/ou quantitative,
et **en ce que** ledit dispositif de transmission (4), pour transmettre via un réseau de télécommunication (R) ladite au moins une image numérique acquise depuis ledit au moins un dispositif de lecture (3) jusqu'audit au moins un dispositif d'analyse (5) situé à distance, comprend :
- des moyens de communication (41) qui sont adaptés à l'échange de données avec ledit dispositif de lecture (3), pour la transmission de ladite au moins une image numérique depuis ledit dispositif de lecture (3) jusqu'audit dispositif de transmission (4), lesquels moyens de communication (33, 41) équipant le dispositif de lecture (3) et le dispositif de transmission (4) consistent en des moyens de connexion sans fils et courte distance,
- des moyens de télécommunication (44), pour la connexion et l'échange de données via le réseau de télécommunication (R), lesquels moyens de télécommunication (44) sont adaptés pour la transmission de ladite au moins une image numérique jusqu'au dispositif d'analyse (5).

2. Système pour l'évaluation qualitative et/ou quantitative d'au moins un analyte, selon la revendication 1, **caractérisé en ce que** le dispositif de transmission (4) comporte un écran (43), et **en ce que** les moyens de fourniture comprennent des moyens pour l'affichage du résultat de ladite évaluation qualitative et/ou quantitative sur ledit écran (43).

3. Système pour l'évaluation qualitative et/ou quantitative d'au moins un analyte, selon l'une quelconque des revendications 1 ou 2, **caractérisé en ce que** le dispositif de transmission (4) consiste en un téléphone mobile, en une tablette numérique ou en un ordinateur.

4. Système pour l'évaluation qualitative et/ou quantitative d'au moins un analyte, selon l'une quelconque des revendications 1 à 3, **caractérisé en ce que** le dispositif d'analyse (5) comprend un programme d'ordinateur comprenant :
- des moyens de code de programme pour la réception de ladite au moins une image numérique depuis ledit dispositif de lecture (3),
- des moyens de code de programme pour traiter ladite au moins une image numérique, tenant compte de données de calibrage,
- des moyens de code de programme pour la fourniture du résultat de ladite évaluation qualitative et/ou quantitative,
lorsque ledit programme d'ordinateur est exécuté par ledit dispositif d'analyse (5).

5. Système pour l'évaluation qualitative et/ou quantitative d'au moins un analyte, selon l'une quelconque des revendications 1 à 4, **caractérisé en ce que** les moyens de réception (31) du dispositif de lecture (3) sont amovibles et interchangeables, de sorte à adapter lesdits moyens de réception (31) en fonction du dispositif immunochromatographique (2) à analyser.

6. Système pour l'évaluation qualitative et/ou quantitative d'au moins un analyte, selon l'une quelconque des revendications 1 à 5, **caractérisé en ce que** le dispositif de lecture (3) comporte des moyens (32) pour la lecture de moyens d'identification (26) portés par le dispositif immunochromatographique (2).

7. Système pour l'évaluation qualitative et/ou quantitative d'au moins un analyte, selon l'une quelconque des revendications 1 à 6, **caractérisé en ce qu'**il comprend encore au moins un dispositif d'affichage distant (6), pour la fourniture du résultat d'évaluation qualitative et/ou quantitative à un tiers distant,
lequel dispositif d'affichage distant (6) est connecté au dispositif d'analyse (5) par le biais du réseau de télécommunication (R), avantageusement Internet,
et **en ce que** les moyens de fourniture du dispositif d'analyse (5) comportent des moyens pour la transmission du résultat d'évaluation audit dispositif d'affichage distant (6) via ledit réseau de télécommunication (R).

8. Système pour l'évaluation qualitative et/ou quantitative d'au moins un analyte, selon l'une quelconque des revendications 1 à 7, **caractérisé en ce qu'**il comprend un dispositif immunochromatographique (2) comprenant :
- une zone amont de libération (22) qui comprend au moins un réactif de détection conjugué avec un marqueur visible et/ou mesurable, ledit réactif de détection étant apte à se déplacer en conséquence de la migration de l'échantillon liquide dans ledit moyen de diffusion capillaire (21), et
- ladite zone aval de révélation (24) qui comprend au moins un réactif de capture, immobilisé sur ledit moyen de diffusion capillaire (21).

9. Système pour l'évaluation qualitative et/ou quantitative d'au moins un analyte, selon l'une quelconque des revendications 1 à 8, **caractérisé en ce qu'**il comprend encore un dispositif de calibrage (7), destiné à être rapporté au niveau des moyens de réception (31) du dispositif de lecture (3) et comportant des données de calibrage destinées à être collectées préalablement à la lecture du dispositif immunochromatographique (2) à analyser, et **en ce que** le dispositif de lecture (3) comporte des moyens pour l'acquisition des données de calibrage portées par ledit dispositif de calibrage (7).

10. Système pour l'évaluation qualitative et/ou quantitative d'au moins un analyte, selon l'une quelconque des revendications 1 à 9, **caractérisé en ce que** le dispositif de lecture (3) comporte encore des moyens (35) pour le raccordement de moyens de communication filaire.

11. Dispositif de lecture (3) constitutif d'un système d'évaluation (1) selon l'une quelconque des revendications précédentes,
lequel dispositif de lecture (3), formant un appareil portatif, comporte :
- des moyens (31) pour la réception dudit dispositif immunochromatographique (2), comportant un emplacement pour le positionnement et le maintien du dispositif immunochromatographique (2),
- des moyens (32) pour l'acquisition d'au moins une image numérique de la zone de révélation (24) dudit dispositif immunochromatographique (2) rapporté dans lesdits moyens de réception (31), lesquels moyens d'acquisition (32) comprennent un capteur photographique,
- des moyens de communication (33) qui sont adaptés à l'échange de données avec au moins un dispositif de transmission (4), pour la transmission de ladite au moins une image numérique depuis ledit dispositif de lecture (3), lesquels moyens de communication (33) consistent en des moyens de connexion sans fils et courte distance,
- des moyens (36) pour l'éclairage de la zone de révélation (24) du dispositif immunochromatographique (2),
- des moyens pour l'alimentation autonome en énergie électrique, comprenant une batterie électrique,
lequel au moins un dispositif de lecture (3) est dépourvu :
- de moyens de traitement de ladite au moins une image numérique, adaptés à l'évaluation qualitative et/ou quantitative dudit au moins un analyte susceptible d'être contenu dans ledit échantillon liquide, et
- de moyens pour la fourniture du résultat d'évaluation qualitative et/ou quantitative.

12. Procédé pour l'évaluation qualitative et/ou quantitative d'au moins un analyte susceptible d'être contenu dans un échantillon liquide déposé sur un dispositif immunochromatographique (2), au moyen d'un système d'évaluation (1) selon l'une quelconque des revendications 1 à 10, lequel procédé comprend les étapes successives suivantes :
- le cas échéant, un appariement dudit dispositif de lecture (3) avec ledit dispositif de transmission (4),
- une mise en place dudit dispositif immunochromatographique (2) au niveau des moyens de réception (31) du dispositif de lecture (3),
- une acquisition de ladite au moins une image numérique de la zone de révélation (24) dudit dispositif immunochromatographique (2) rapporté dans lesdits moyens de réception (31),
- une transmission de ladite au moins une image numérique depuis le dispositif de lecture (3) jusqu'au dispositif de transmission (4), par le biais des moyens de communication (33, 14),
- une transmission de ladite au moins une image numérique depuis ledit dispositif de transmission (4) jusqu'audit dispositif d'analyse (5), via ledit réseau de télécommunication (R),
- un traitement de ladite au moins une image numérique par ledit dispositif d'analyse (5), pour l'évaluation qualitative et/ou quantitative dudit au moins un analyte susceptible d'être contenu dans ledit échantillon liquide, et
- une fourniture du résultat de ladite évaluation qualitative et/ou quantitative.

## Patentansprüche

1. System zur Auswertung wenigstens eines Analyten, der in einer auf einer immunochromatographischen Vorrichtung (2) abgelegten flüssigen Probe enthalten sein kann,
wobei die immunochromatographische Vorrichtung (2) kapillare Diffusionsmittel (21) aufweist, auf denen die flüssige Probe von einer davor liegenden Ablagezone (22) zu einer danach liegenden Offenbarungszone (24) wandern kann,
**dadurch gekennzeichnet, daß** das Auswertungssystem (1) wenigstens eine Lesevorrichtung (3), eine Übertragungsvorrichtung (4) und wenigstens eine Analysevorrichtung (5) aufweist, die sich von der Struktur her voneinander unterscheiden,
daß die ein tragbares Gerät bildende wenigstens eine Lesevorrichtung (3)
- Mittel (31) zum Aufnehmen der immunochromatographischen Vorrichtung (2), die einen Platz zum Positionieren und Festhalten der immunochromatographischen Vorrichtung (2) aufweisen,
- in die Aufnahmemittel (31) eingebrachte Mittel (32) zum Erfassen wenigstens eines digitalen Bilds der Offenbarungszone (24) der immunochromatographischen Vorrichtung (2), wobei die Erfassungsmittel (32) einen fotografischen Sensor aufweisen,
- Kommunikationsmittel (33), die zum Austausch von Daten mit wenigstens der Übertragungsvorrichtung (4) zum Übertragen des wenigstens einen digitalen Bilds von der Lesevorrichtung (3) aus ausgelegt sind,
- Mittel (36) zum Beleuchten der Offenbarungszone (24) der immunochromatographischen Vorrichtung (2),
- Mittel für die autonome Versorgung mit elektrischer Energie, die eine elektrische Batterie aufweisen,
aufweist,
wobei die wenigstens eine Lesevorrichtung (3) keine
- Mittel zum Bearbeiten des wenigstens einen digitalen Bilds, die zur qualitativen und/oder quantitativen Auswertung des wenigstens einen Analyten, der in der flüssigen Probe enthalten sein kann, ausgelegt sind, und keine
- Mittel zum Bereitstellen des Ergebnisses der qualitativen und/oder quantitativen Auswertung
aufweist,
daß die wenigstens eine Analysevorrichtung (5)
- Kommunikationsmittel (52), die für den Datenaustausch, zum Empfangen des wenigstens einen digitalen Bilds, ausgelegt sind, wobei die Kommunikationsmittel (52) der Analysevorrichtung (5) in Form von Fernmeldemitteln für die Verbindung und den Datenaustausch über das Fernmeldenetz (R) vorliegen,
- Mittel (51) zum Bearbeiten des wenigstens einen digitalen Bilds, die zur qualitativen und/oder quantitativen Auswertung des wenigstens einen Analyten, der in der flüssigen Probe enthalten sein kann, ausgelegt sind, und
- Mittel zum Bereitstellen des Ergebnisses der qualitativen und/oder quantitativen Auswertung
aufweist,
und daß die Übertragungsvorrichtung (4) zum Übertragen des wenigstens einen erfaßten digitalen Bilds von der wenigstens einen Lesevorrichtung (3) zur entfernt gelegenen Analysevorrichtung (5) über ein Fernmeldenetz (R)
- Kommunikationsmittel (41), die für den Datenaustausch mit der Lesevorrichtung (3), für die Übertragung des wenigstens einen digitalen Bilds von der Lesevorrichtung (3) zur Übertragungsvorrichtung (4) ausgelegt sind, wobei die Kommunikationsmittel (33, 41), mit denen die Lesevorrichtung (3) und die Übertragungsvorrichtung (4) ausgestattet sind, aus drahtlosen Verbindungsmitteln für kurze Strecken bestehen,
- Fernmeldemittel (44) für die Verbindung und den Datenaustausch über das Fernmeldenetz (R), wobei die Fernmeldemittel (44) für die Übertragung des wenigstens einen digitalen Bilds zur Analysevorrichtung (5) ausgelegt sind, aufweist.

2. System zur qualitativen und/oder quantitativen Auswertung wenigstens eines Analyten gemäß Anspruch 1, **dadurch gekennzeichnet, daß** die Übertragungsvorrichtung (4) einen Bildschirm (43) aufweist und daß die Bereitstellungsmittel Mittel zum Anzeigen des Ergebnisses der qualitativen und/oder quantitativen Auswertung auf dem Bildschirm (43) aufweisen.

3. System zur qualitativen und/oder quantitativen Auswertung wenigstens eines Analyten gemäß einem der Ansprüche 1 oder 2, **dadurch gekennzeichnet, daß** die Übertragungsvorrichtung (4) aus einem tragbaren Telefon, einem Tablet-Computer oder einem Computer besteht.

4. System zur qualitativen und/oder quantitativen Auswertung wenigstens eines Analyten gemäß einem der Ansprüche 1 bis 3, **dadurch gekennzeichnet, daß** die Analysevorrichtung (5) ein Computerprogramm aufweist, das
- Programmcodemittel zum Empfangen des wenigstens einen digitalen Bilds von der Lesevorrichtung (3),
- Programmcodemittel zum Bearbeiten des wenigstens einen digitalen Bilds unter Berücksichtigung von Kalibrierdaten,
- Programmcodemittel zum Bereitstellen des Ergebnisses der qualitativen und/oder quantitativen Auswertung
aufweist,
wenn das Computerprogramm von der Analysevorrichtung (5) durchgeführt wird.

5. System zur qualitativen und/oder quantitativen Auswertung wenigstens eines Analyten gemäß einem der Ansprüche 1 bis 4, **dadurch gekennzeichnet, daß** die Aufnahmemittel (31) der Lesevorrichtung (3) abnehmbar und austauschbar sind, um die Aufnahmemittel (31) in Abhängigkeit von der zu analysierenden immunochromatographischen Vorrichtung (2) anzupassen.

6. System zur qualitativen und/oder quantitativen Auswertung wenigstens eines Analyten gemäß einem der Ansprüche 1 bis 5, **dadurch gekennzeichnet, daß** die Lesevorrichtung (3) Mittel (32) zum Lesen von von der immunochromatographischen Vorrichtung (2) getragenen Identifizierungsmitteln (26) aufweist.

7. System zur qualitativen und/oder quantitativen Auswertung wenigstens eines Analyten gemäß einem der Ansprüche 1 bis 6, **dadurch gekennzeichnet, daß** es außerdem wenigstens eine entfernte Anzeigevorrichtung (6) zum Bereitstellen des Ergebnisses der qualitativen und/oder quantitativen Auswertung bei einem entfernten Dritten aufweist,
wobei die entfernte Anzeigevorrichtung (6) über das Fernmeldenetz (R), vorzugsweise über Internet, mit der Analysevorrichtung (5) verbunden ist,
und daß die Bereitstellungsmittel der Analysevorrichtung (5) Mittel zum Übertragen des Auswertungsergebnisses über das Fernmeldenetz (R) an die entfernte Anzeigevorrichtung (6) aufweisen.

8. System zur qualitativen und/oder quantitativen Auswertung wenigstens eines Analyten gemäß einem der Ansprüche 1 bis 7, **dadurch gekennzeichnet, daß** es eine immunochromatographische Vorrichtung (2) mit
- einer davor liegenden Ablagezone (22), die wenigstens ein mit einem sichtbaren und/oder meßbaren Marker versetztes Nachweisreagens aufweist, wobei das Nachweisreagens in der Lage ist, sich entsprechend dem Wandern der flüssigen Probe im kapillaren Diffusionsmittel (21) zu bewegen, und
- der danach liegenden Offenbarungszone (24), die wenigstens ein auf dem kapillaren Diffusionsmittel (21) festgemachtes Fangreagens aufweist.

9. System zur qualitativen und/oder quantitativen Auswertung wenigstens eines Analyten gemäß einem der Ansprüche 1 bis 8, **dadurch gekennzeichnet, daß** es außerdem eine Kalibriervorrichtung (7) aufweist, die dazu bestimmt ist, im Bereich der Aufnahmemittel (31) der Lesevorrichtung (3) eingefügt zu werden, und Kalibrierdaten enthält, die dazu bestimmt sind, vor dem Auslesen der zu analysierenden immunochromatographischen Vorrichtung (2) gesammelt zu werden, und daß die Lesevorrichtung (3) Mittel zum Erfassen der von der Kalibriervorrichtung (7) getragenen Kalibrierdaten aufweist.

10. System zur qualitativen und/oder quantitativen Ausweitung wenigstens eines Analyten gemäß einem der Ansprüche 1 bis 9, **dadurch gekennzeichnet, daß** die Lesevorrichtung (3) außerdem Mittel (35) zum Anschließen von drahtgebundenen Kommunikationsmitteln aufweist.

11. Lesevorrichtung (3), die ein Auswertesystem (1) gemäß einem der vorangehenden Ansprüche bildet,
wobei die ein tragbares Gerät bildende Lesevorrichtung (3)
- Mittel (31) zum Aufnehmen der immunochromatographischen Vorrichtung (2), die einen Platz zum Positionieren und Festhalten der immunochromatographischen Vorrichtung (2) aufweisen,
- in die Aufnahmemittel (31) eingebrachte Mittel (32) zum Erfassen wenigstens eines digitalen Bilds der Offenbarungszone (24) der immunochromatographischen Vorrichtung (2), wobei die Erfassungsmittel (32) einen fotografischen Sensor aufweisen,
- Kommunikationsmittel (33), die zum Austausch von Daten mit wenigstens einer Übertragungsvorrichtung (4) zum Übertragen des wenigstens einen digitalen Bilds von der Lesevorrichtung (3) aus ausgelegt sind, wobei die Kommunikationsmittel (33) aus Mitteln zur drahtlosen Verbindung über kurze Entfernung bestehen,
- Mittel (36) zum Beleuchten der Offenbarungszone (24) der immunochromatographischen Vorrichtung (2),
- Mittel für die autonome Versorgung mit elektrischer Energie, die eine elektrische Batterie aufweisen,
aufweist,
wobei die wenigstens eine Lesevorrichtung (3) keine
- Mittel zum Bearbeiten des wenigstens einen digitalen Bilds, die zur qualitativen und/oder quantitativen Auswertung des wenigstens einen Analyten, der in der flüssigen Probe enthalten sein kann, ausgelegt sind, und keine
- Mittel zum Bereitstellen des Ergebnisses der qualitativen und/oder quantitativen Auswertung
aufweist.

12. Verfahren zur qualitativen und/oder quantitativen Auswertung wenigstens eines Analyten, der in einer Flüssigkeitsprobe enthalten sein kann, auf einer immunochromatographischen Vorrichtung (2) mittels eines Auswertungssystems (1) gemäß einem der Ansprüche 1 bis 10, wobei das Verfahren die folgenden aufeinanderfolgenden Schritte aufweist:
- gegebenenfalls ein Abstimmen der Lesevorrichtung (3) mit der Übertragungsvorrichtung (4),
- ein Einsetzen der immunochromatographischen Vorrichtung (2) im Bereich der Aufnahmemittel (31) der Lesevorrichtung (3),
- ein Erfassen des wenigstens einen digitalen Bilds der Offenbarungszone (24) der in die Aufnahmemittel (31) eingebrachten immunochromatographischen Vorrichtung (2),
- eine Übertragung des wenigstens einen digitalen Bilds von der Lesevorrichtung (3) bis zur Übertragungsvorrichtung (4) mittels Kommunikationsmitteln (33, 14),
- eine Übertragung des wenigstens einen digitalen Bilds von der Übertragungsvorrichtung (4) bis zur Auswertungsvorrichtung (5) über das Fernmeldenetz (R),
- eine Bearbeitung des wenigstens einen digitalen Bilds durch die Analysevorrichtung (5) zur qualitativen und/oder quantitativen Auswertung des wenigstens einen Analyten, der in der flüssigen Probe enthalten sein kann, und
- ein Bereitstellen des Ergebnisses der qualitativen und/oder quantitativen Auswertung.

## Claims

1. A system for qualitative and/or quantitative evaluation of at least one analyte likely to be contained in a liquid sample deposited on an immunochromatographic device (2),
wherein said immunochromatographic device (2) includes capillary diffusion means (21) on which said liquid sample is intended to migrate from an upstream deposition zone (22) to a downstream revelation zone (24),
**characterized in that** said evaluation system (1) comprises at least one reading device (3), a transmission device (4) and at least one analysis device (5), which are structurally distinct from each other,
and **in that** said at least one reading device (3), forming a portable apparatus, includes:
- means (31) for receiving said immunochromatographic device (2), including a location for the positioning and holding of the immunochromatographic device (2),
- means (32) for acquiring at least one digital image of the revelation zone (24) of said immunochromatographic device (2) placed into said reception means (31), said acquisition means (32) comprising a photographic sensor,
- communication means (33) adapted for data exchange with at least the transmission device (4), for the transmission of said at least one digital image from said reading device (3),
- means (36) for lighting the revelation zone (24) of the immunochromatographic device (2),
- electric power self-supply means, comprising an electric battery,
said at least one reading device (3) being devoid of:
- means for processing said at least one digital image, adapted to the qualitative and/or quantitative evaluation of said at least one analyte likely to be contained in said liquid sample, and
- means for providing the qualitative and/or quantitative evaluation result,
**in that** said at least one analysis device (5) comprises:
- communication means (52) adapted for data exchange, for the reception of said at least one digital image, said communication means (52) of the analysis device (5) being in the form of telecommunications means, for connection and data exchange via the telecommunications network (R),
- means (51) for processing said at least one digital image, adapted to the qualitative and/or quantitative evaluation of said at least one analyte likely to be contained in said liquid sample, and
- means for providing the result of said qualitative and/or quantitative evaluation,
and **in that** said transmission device (4), for transmitting said at least one acquired digital image, via a telecommunications network (R), from said at least one reading device (3) to at least one remote analysis device (5), comprises:
- communication means (41) adapted for data exchange with said reading device (3), for the transmission of said at least one digital image from said reading device (3) to said transmission device (4), wherein said communication means (33, 41) equipping the reading device (3) and the transmission device (4) consist of short-range wireless connection means,
- telecommunications means (44), for connection and data exchange via the telecommunications network (R), wherein said telecommunications means (44) are adapted for the transmission of said at least one digital image to the analysis device (5).

2. The system for qualitative and/or quantitative evaluation of at least one analyte according to claim 1, **characterized in that** the transmission device (4) includes a screen (43), and **in that** the provision means comprise means for displaying the result of said qualitative and/or quantitative evaluation on said screen (43).

3. The system for qualitative and/or quantitative evaluation of at least one analyte according to any one of claims 1 or 2, **characterized in that** the transmission device (4) consists of a mobile phone, a digital tablet or a computer.

4. The system for qualitative and/or quantitative evaluation of at least one analyte according to any one of claims 1 to 3, **characterized in that** the analysis device (5) comprises a computer program comprising:
- program code means for receiving said at least one digital image from said reading device (3),
- program code means for processing said at least one digital image, considering calibration data,
- program code means for providing the result of said qualitative and/or quantitative evaluation,
when said computer program is executed by said analysis device (5).

5. The system for qualitative and/or quantitative evaluation of at least one analyte according to any one of claims 1 to 4, **characterized in that** the reception means (31) of the reading device (3) are removable and interchangeable, in such a way as to adapt said reception means (31) as a function of the immunochromatographic device (2) to be analysed.

6. The system for qualitative and/or quantitative evaluation of at least one analyte according to any one of claims 1 to 5, **characterized in that** the reading device (3) includes means (32) for reading identification means (26) carried by the immunochromatographic device (2).

7. The system for qualitative and/or quantitative evaluation of at least one analyte according to any one of claims 1 to 6, **characterized in that** it still comprises at least one remote display device (6), for providing the qualitative and/or quantitative evaluation result to a remote third party,
said remote display device (6) being connected to the analysis device (5) through the telecommunications network (R), advantageously Internet,
and **in that** the provision means of the analysis device (5) include means for transmitting the evaluation result to said remote display device (6) via said telecommunications network (R).

8. The system for qualitative and/or quantitative evaluation of at least one analyte according to any one of claims 1 to 7, **characterized in that** it comprises an immunochromatographic device (2) comprising:
- an upstream release zone (22) that comprises at least one detection reagent conjugated with a visible and/or measurable marker, said detection reagent being adapted to move due to the migration of the liquid sample in said capillary diffusion means (21), and
- said downstream revelation zone (24) that comprises at least one capture reagent, immobilized on said capillary diffusion means (21).

9. The system for qualitative and/or quantitative evaluation of at least one analyte according to any one of claims 1 to 8, **characterized in that** it also comprises a calibration device (7), intended to be placed at the reception means (31) of the reading device (3) and including calibration data intended to be collected previously to the reading of the immunochromatographic device (2) to be analysed, and **in that** the reading device (3) includes means for acquiring calibration data carried by said calibration device (7).

10. The system for qualitative and/or quantitative evaluation of at least one analyte according to any one of claims 1 to 9, **characterized in that** the reading device (3) also includes means (35) for the connection of wired communication means.

11. A reading device (3) constituent of an evaluation system (1) according to any one of the preceding claims,
wherein said reading device (3), forming a portable apparatus, includes:
- means (31) for receiving said immunochromatographic device (2), including a location for the positioning and holding of the immunochromatographic device (2),
- means (32) for acquiring at least one digital image of the revelation zone (24) of said immunochromatographic device (2) placed into said reception means (31), said acquisition means (32) comprising a photographic sensor,
- communication means (33) adapted for data exchange with at least one transmission device (4), for the transmission of said at least one digital image from said reading device (3), wherein said communication means (33) consist of short-range wireless connection means,
- means (36) for lighting the revelation zone (24) of the immunochromatographic device (2),
- electric power self-supply means, comprising an electric battery,
said at least one reading device (3) being devoid of:
- means for processing said at least one digital image, adapted to the qualitative and/or quantitative evaluation of said at least one analyte likely to be contained in said liquid sample, and
- means for providing the qualitative and/or quantitative evaluation result.

12. A method for the qualitative and/or quantitative evaluation of at least one analyte likely to be contained in a liquid sample deposited on an immunochromatographic device (2), by means of an evaluation system (1) according to any one of claims 1 to 10, wherein said method comprises the following successive steps:
- as the case may be, matching said reading device (3) with said transmission device (4),
- positioning said immunochromatographic device (2) at the reception means (31) of the reading device (3),
- acquiring said at least one digital image of the revelation zone (24) of said immunochromatographic device (2) placed in said reception means (31),
- transmitting said at least one digital image from the reading device (3) to the transmission device (4), through communication means (33, 14),
- transmitting said at least one digital image from said transmission device (4) to said analysis device (5), via said telecommunications means (R),
- processing said at least one digital image by said analysis device (5), for the qualitative and/or quantitative evaluation of said at least one analyte likely to be contained in said liquid sample, and
- providing the result of said qualitative and/or quantitative evaluation.
